Europäisches Patentamt

European Patent Office

Office européen des brevets

(19)

(11) **EP 1 273 296 B1**

(12) **EUROPEAN PATENT SPECIFICATION**

(45) Date of publication and mention
of the grant of the patent:
**26.11.2003 Bulletin 2003/48**

(51) Int Cl.⁷: **A61K 31/337**, A61K 31/166,
A61K 9/00, A61P 35/00
// (A61K31/337, 31:166)

(21) Application number: **02254337.5**

(22) Date of filing: **01.07.2002**

(54) **COMBINATION OF ACETYLDINALINE AND DOCETAXEL**

KOMBINATION VON ACETYLDINALIN UND DOCETAXEL

COMBINAISON DE L'ACETYLDINALINE ET DE DOCETAXEL

(84) Designated Contracting States:
**AT BE BG CH CY CZ DE DK EE ES FI FR GB GR
IE IT LI LU MC NL PT SE SK TR**

(30) Priority: **02.07.2001 US 302415 P**

(43) Date of publication of application:
**08.01.2003 Bulletin 2003/02**

(73) Proprietor: **WARNER-LAMBERT COMPANY
Morris Plains, New Jersey 07950 (US)**

(72) Inventors:
• **Grove, William Richard
Ann Arbor, Michigan 48105 (US)**
• **Merriman, Ronald Lynn
Ann Arbor, Michigan 48105 (US)**

(74) Representative: **England, Peter Michael
Pfizer Limited,
UK Patent Department,
Ramsgate Road
Sandwich, Kent CT13 9NJ (GB)**

(56) References cited:
**WO-A-00/18393** **WO-A-01/34131**
**US-A- 5 137 918**

• **LORUSSO P M ET AL: "PRECLINICAL
ANTITUMOR ACTIVITY OF CI-994"
INVESTIGATIONAL NEW DRUGS, MARTINUS
NIJHOFF PUBLISHERS, BOSTON, US, vol. 14,
no. 4, 1996, pages 349-356, XP002071743 ISSN:
0167-6997**
• **BISSERY M-C ET AL: "PRECLINICAL PROFILE
OF DOCETAXEL (TAXOTERE): EFFICACY AS A
SINGLE AGENT AND IN COMBINATION"
SEMINARS IN ONCOLOGY, BETHESDA, MD, US,
vol. 22, no. 6, SUPPL 13, 1 December 1995
(1995-12-01), pages 3-16, XP002065512**

EP 1 273 296 B1

**Description**

FIELD OF THE INVENTION

The invention concerns the use of a combination of known oncolytic agents for the manufacture of a medicament for the treatment of tumors. The use of the agents together provides unexpectedly greater efficacy than employing the single agents alone.

BACKGROUND OF THE INVENTION

Cancer chemotherapy has advanced dramatically in recent years. Many tumors can be effectively treated utilizing compounds which are either naturally occurring products or synthetic agents. Cancer chemotherapy often entails use of a combination of agents, generally as a means of providing greater therapeutic effects and reducing the toxic effects that are often encountered with the individual agents when used alone.

We have now discovered a unique combination of known oncolytic agents which exhibits a dramatic synergistic effect. The combination utilizes the agent acetyldinaline, together with docetaxel. The combination is especially effective in treating patients with solid tumors, especially nonsmall cell lung cancer and other advanced solid tumors.

Acetyldinaline is 4-acetylamino-N-(2'-aminophenyl)-benzamide. It is also known as CI-994. It is described in United States Patent Number 5,137,918, how to make acetyldinaline, how to formulate it into dosage forms, and how to use it for treating cancers such as colon cancer and adenocarcinomas. Acetyldinaline is also described in United States Patent Number 5,795,909 as a possible conjugate for cancer treatment.

Docetaxel is a semi-synthetic compound belonging to the taxoid family. It is an antimicrotubule agent that promotes the assembly of micro tubules from tubulin dimers and stabilizes microtubules by preventing depolymerization. This stability results in the inhibition of the normal dynamic reorganization of the microtubule network that is essential for vital interphase and mitotic cellular functions. In addition, docetaxel induces abnormal arrays or bundles of microtubules throughout the cell cycle and multiple asters of microtubules during mitosis. Docetaxel is indicated primarily for breast cancer and non-small cell lung cancer, although it is useful in treating other cancers as well. Use of docetaxel is generally accompanied by undesirable side effects, including hypersensitivity reactions, hypotension, bradycardia, hypertension, nausea and vomiting, and injection site reactions. Docetaxel trihydrate is commercially available as Taxotere® (Aventis Phamaceutical Products, Inc., Collegeville, PA).

An object of this invention is to provide, a combination comprising acetyldinaline together with docetaxel for the manufacture of a medicament for the treatment of cancer, especially advanced solid tumors. A further object is to provide a composition comprising synergistic amounts of acetyldinaline and docetaxel.

SUMMARY OF THE INVENTION

This invention relates to a synergistic combination of antineoplastic agents, and to the use of the combination for the manufacture of a medicament for the treatment of tumors. The invention more particularly provides a composition comprising, as a first component, acetyldinaline, and as a second component, docetaxel.

The compositions of this invention consist essentially of the above active ingredients, or suitable salts thereof, together with common excipients, diluents, and carriers.

In a further embodiment of the invention, we provide the use of a combination of acetyldinaline with docetaxel for the manufacture of a medicament for the treatment of cancer.

A preferred use embraces treatment of solid tumors.

A further preferred use employs an antitumor amount of acetyldinaline and an effective amount of docetaxel to treat susceptible cancers, including NSCLC, breast cancer, ovarian cancer, head and neck cancer, myelomas, prostate cancer, and pancreatic cancer.

Another embodiment of the invention is a kit comprising in one compartment a dosage of acetyldinaline, and in another compartment a dosage of docetaxel.

According to a further aspect of the present invention, there are provided products comprising acetyldinaline or a pharmaceutically acceptable salt or hydrate thereof and docetaxel or a pharmaceutically acceptable salt or hydrate thereof for simultaneous, separate or sequential use in the curative, palliative or prophylactic treatment of cancer.

Preferably, there are provided products comprising acetyldinaline or a pharmaceutically acceptable salt or hydrate thereof and docetaxel or a pharmaceutically acceptable salt or hydrate thereof as a combined preparation for simultaneous, separate or sequential use in the curative, palliative or prophylactic treatment of cancer.

Alternatively, there are provided products comprising, either separately or together, acetyldinaline or a pharmaceutically acceptable salt or hydrate thereof and docetaxel or a pharmaceutically acceptable salt or hydrate thereof for simultaneous, separate or sequential use in the curative, palliative or prophylactic treatment of cancer.

Preferably, there are provided products comprising, either separately or together, acetyldinaline or a pharmaceutically acceptable salt or hydrate thereof and docetaxel or a pharmaceutically acceptable salt or hydrate thereof as a combined preparation for simultaneous, separate or sequential use in the curative, palliative or prophylactic treatment of cancer.

According to yet a further aspect of the present invention, there are provided products containing acetyldinaline or a pharmaceutically acceptable salt or hydrate thereof and docetaxel or a pharmaceutically acceptable salt or hydrate thereof for simultaneous, separate or sequential use in the curative, palliative or prophylactic treatment of cancer.

Preferably, there are provided products containing acetyldinaline or a pharmaceutically acceptable salt or hydrate thereof and docetaxel or a pharmaceutically acceptable salt or hydrate thereof as a combined preparation for simultaneous, separate or sequential use in the curative, palliative or prophylactic treatment of cancer.

Alternatively, there are provided products containing, either separately or together, acetyldinaline or a pharmaceutically acceptable salt or hydrate thereof and docetaxel or a pharmaceutically acceptable salt or hydrate thereof for simultaneous, separate or sequential use in the curative, palliative or prophylactic treatment of cancer.

Preferably, there are provided products containing, either separately or together, acetyldinaline or a pharmaceutically acceptable salt or hydrate thereof and docetaxel or a pharmaceutically acceptable salt or hydrate thereof as a combined preparation for simultaneous, separate or sequential use in the curative, palliative or prophylactic treatment of cancer.

The terms product(s), medicament(s), composition(s), combination(s), pharmaceutical(s), pharmaceutical composition(s) and pharmaceutical combination(s) are, in the context of the present invention, to be taken as having equivalent meanings.

DETAILED DESCRIPTION OF THE INVENTION

The compounds to be utilized in the method of this invention will be administered in doses commonly employed clinically. Such doses will be calculated in the normal fashion, for example on body surface area. Acetyldinaline will be administered, for example, at doses from about 1.0 mg/m$^2$ to about 50 mg/m$^2$, preferably from about 2.0 mg/m$^2$ to about 10.0 mg/m$^2$. Ideally, acetyldinaline will be administered at a dose which will produce plasma levels of about 5 to about 100 $\mu$g/mL. Acetyldinaline typically is administered orally, for example, as capsules having active ingredient in the amounts of 2.5, 5, and 25 mg per capsule. Acetyldinaline will be administered daily at about the same dose levels throughout a treatment period, typically for 15 to 30 days. Multiple treatment periods can be practiced, as dictated by the attending medical practitioner and the particular patient and condition being treated.

Docetaxel is a cytotoxic anticancer drug, and caution should be exercised in handling the agent. Docetaxel typically is provided in vials, and is diluted prior to administration by intravenous infusion. Typical diluents include 0.9% sodium chloride, and 5% dextrose. The final concentration for infusion liquid generally is about 0.3 to about 1.2 mg/mL. Docetaxel is commercially available in several concentrations, for instance, 20 mg/0.5 mL concentrate; 80 mg/2.0 mL concentrate. The product generally is administered, for treatment of breast cancer for example, at doses of about 60 mg/m$^2$ to about 100 mg/m$^2$ over 1 to 2 hours every 3 weeks.

The agent is an effective treatment for carcinoma of the breast at doses of about 100 mg/m$^2$ administered IV over about 1 hour every 3 weeks. For treatment of AIDS-related Kaposi's sarcoma, docetaxel generally is given IV at about 75 mg/m$^2$ over 1 to 2 hours every 3 weeks, or at 100 mg/m$^2$ over 3 hours every 2 weeks. In general, the dosage intensity of docetaxel will be about 45 to 50 mg/m$^2$/week.

In a preferred embodiment, a typical invention combination will be administered at the following doses shown in Table 1.

Table 1

| Docetaxel Dose (mg/m$^2$ infused over 1 hour on Day 1) | Acetyldinaline Dose (oral doses administered daily on Days 1-14) |
|---|---|
| 60 | 2.5 mg fixed dose |
| 80 | 5 mg fixed dose |
| 100 | 6 mg/m$^2$/day |
| 120 | 4 mg/m$^2$/day |

Another typical dosing embodiment is shown in Table 2.

Table 2

| Docetaxel Dose<br>(mg infused over 30 minutes on Day 1) | Acetyldinaline Dose (orally dosed each day for Days 1-14) |
| --- | --- |
| 50 | 2.5 mg fixed dose |
| 75 | 6 mg fixed dose |
| 80 | 6 mg/m$^2$/day |
| 100 | 4 mg/m$^2$/day |

The combinations provided by this invention have been evaluated in several assay systems, and the data can be analyzed utilizing a standard program for quantifying synergism, additivism, and antagonism among anticancer agents. The program preferably utilized is that described by Chou and Talalay in "New Avenues in Developmental Cancer Chemotherapy," *Academic Press,* 1987, Chapter 2.

The method is based on the median-effect principle of the mass-action law using an enzyme kinetic system as a model. The equation is simple and describes the relationships between dose and effect regardless of the shape of the dose-effect curve. Two basic equations constitute the pillars of this methodology. To relate dose and effect for a single drug in the simplest way possible, the median-effect equation derived by Chou is given by:

$$f_a/f_u = (D/D_m)^m$$

$$D = D_m[f_a/(1-f_a)]^{1/m}$$

where the right side represents the dose and the left side represents the effect, in which $f_a$ and $f_u$ are the fractions affected and unaffected, respectively, D is the dose, $D_m$ is the median-effect dose signifying the potency, and m is a coefficient signifying the shape of the dose-effect curve. From this equation Chou and Talalay derived the general equation for two or more drugs:

$$\left[\frac{(f_a)_{1,2}}{(f_u)_{1,2}}\right]^{1/m} = \left[\frac{(f_a)_1}{(f_u)_1}\right]^{1/m} + \left[\frac{(f_a)_2}{(f_u)_2}\right]^{1/m} = +\alpha\left[\frac{(f_a)_1 (f_a)_2}{(f_u)_1 (f_u)_2}\right]^{1/m}$$

$$= \frac{(D)_1}{(D_m)_1} + \frac{(D)_2}{(D_m)_1} = \frac{\alpha (D)_1 (D)_2}{(D_m)_1 (D_m)_2}$$

where m = 1 is for first-order Michaelis-Menten-type kinetics and m >1 (or m <1) is for higher order (or lower order) Hill-type kinetics. When alpha = 0, the third term on the right side disappears and when alpha = 1, the third term is conserved. Alpha = 0 is used for mutually exclusive drugs and alpha = 1 is used for mutually nonexclusive drugs. For drugs that have the same or similar modes of action, the effects of both drugs are mutually exclusive. For drugs that have different modes of action or act independently, the effects of both drugs are mutually nonexclusive.

A plot of fraction affected ($F_a$) versus combination index (CI) is called the $F_a$-CI plot. This plot indicates synergism, additivity, or antagonism of two drugs at various effect levels in a mixture that is serially diluted. If several mixtures are made, it is possible to estimate the optimal combination ratio for maximal synergy. Different effect levels usually give different degress of synergism, additivism, or antagonism. CI values <1 indicate synergism; CI values >1 indicate antagonism, and CI values that are one or hover around one indicate additivity. For anticancer agents, synergism at high effect levels ($F_a$) is clinically more relevant than synergism at low $F_a$ levels.

While acetyldinaline (CI-994) has not been approved for clinical use, it has nevertheless been evaluated in several clinical trials. In one such study, patients were treated using a dose-escalation scheme that increased both the daily dose and the duration of treatment. The majority of patients had received extensive prior chemotherapy. The maximum-tolerated dose (MTD) was 15 mg/m$^2$/day when the duration of treatment was 14 consecutive days. To allow more prolonged treatment, lower doses were studied. Using a schedule of 8 weeks of continuous daily therapy, followed by a 2-week 'drug holiday', the MTD was 8 mg/m$^2$/day. The dose-limiting toxicity was thrombocytopenia or neutropenia, usually occurring within 1 month of the start of therapy. Blood counts tended to stabilize even with continued treatment and to recover quickly when treatment was stopped. There was no evidence of cumulative toxicity following repeated courses and prolonged exposures to CI-994. Other toxicities included nausea, vomiting, diarrhea, anorexia, fatigue, mucositis, headache, dehydration, and increases in liver and renal function test values. Responses included one partial response in a heavily pretreated patient with NSCLC and a minor response in one patient each with renal cell cancer and NSCLC.

An additional Phase 1 study was conducted in patients with relapsed acute leukemia or other hematologic malignancy using a once daily high-dose 5-day dosing schedule. The MTD was 135 mg/m$^2$/day. The dose-limiting toxicity was acute CNS toxicity manifested as sedation and somnolence. Other adverse events included nausea, vomiting, hypotension resulting from dehydration, hypocalcemia, headache, and in one patient each, acute pancreatitis, a pyramidal syndrome characterized by hyperreflexia and bilateral Babinski reflexes, and sepsis. Hematologic toxicities cannot be assessed in this patient population. Two patients with AML developed tumor lysis syndrome, resulting in one death. Transient decreases in peripheral white blood cell counts were noted.

A Phase 2 program is currently being conducted with CI-994, used as a single agent. The dosing regimen is 8 mg/m$^2$ given orally daily. Over 100 patients have been treated, including patients with nonsmall cell lung cancer, renal cell cancer, pancreatic cancer, head and neck cancer, ovarian cancer, myeloma, prostate cancer, and breast cancer. Some patients have tolerated dose increases to 10 mg/m$^2$, while some patients have had to have treatment interrupted due to thrombocytopenia, and then be restarted on CI-994 at lowered doses. The adverse events have been similar to those observed in the chronic dosing Phase 1 protocol. Thrombocytopenia has been the dose-limiting toxicity. Infrequent neurologic adverse events including paresthesias, confusion, and hallucinations have been reported. Objective responses have been seen in patients with nonsmall cell lung cancer. Clinical benefit has been reported in patients with renal cell cancer.

In the solid tumor Phase 1 study, CI-994 doses were administered orally following a fasting period, and blood samples were collected for pharmacokinetic analyses. Preliminary results indicate that the maximum blood level is achieved approximately 1 to 2 hours after ingestion, and the terminal elimination half-life of CI-994 is approximately 15 hours. The maximum plasma CI-994 concentrations achieved with increasing dose levels were less than dose-proportional. The terminal elimination half-life and the apparent clearance rate were independent of the dose administered.

One additional objective of this study was to determine whether taking CI-994 with food affected its rate or degree of absorption. Twelve fasted patients were given a single dose of CI-994, 8 mg/m$^2$. One week later, the same patients were given the same dose of CI-994 with a normal meal. Analysis of pharmacokinetic data revealed that CI-994 can be taken without regard to meals.

Mass balance/route of elimination studies have not been conducted in humans. Animal studies indicate that the principal route of elimination is via renal excretion, with 80% and 62% of radiolabeled drug appearing in the urine of monkeys and rats, respectively, within 24 hours.

The following detailed examples further establish the synergy between CI-994 and docetaxel.

EXAMPLE 1

The synergistic combinations provided by this invention have been evaluated in standard chemotherapy studies using female BALB/C mice weighing 18 to 20 g. The test mice were obtained from Charles River Laboratories, Wilmington, MA. On Day 0 of the test, each mouse was surgically implanted (subcutaneously) with a fragment of LC-12 squamous cell lung carcinoma tumor weighing approximately 30 to 60 mg. The tumor fragments were implanted using a 12 gauge trocar. The mice were weighed weekly, and tumor size (width and length in mm) were measured two times each week with standard calipers. Tumor mass for each animal was calculated according to the formula:

$$\text{Tumor weight (mg)} = \frac{(a \times b^2)}{2},$$

where "a" is width of the tumor in mm, and "b" is the length in mm. Evaluation of anticancer activity was established by the formula T-C, where "T" and "C" are the median time (in days) required for the treated and control (respectively) tumors to reach a predetermined size of about 750 mg (the "evaluation size"). Tumors generally reached size of about 150 to about 200 mg before drug dosing was initiated. Antitumor activity was assessed according to four parameters: (1) partial tumor response (PR); (2) complete tumor response (CR); (3) tumor-free survival (TF); and (4) tumor growth delay (TL). Tumor growth delay is expressed as a T-C value, where T is the median days required for the treatment group tumors to reach a predetermined size (e.g., 750 mg), and C is the median days for the control group tumors to reach this size. From the tumor growth delay value, the net $\log_{10}$ tumor cell kill is calculated as follows:

$$\text{Net } \log_{10} \text{ tumor cell kill} = [(T\text{-}C) - Rx]/3.32 \times Td$$

where Td is the days for the tumor mass to double, and Rx is the total days of treatment. Td is estimated from the best fit straight line from a log-linear plot of the control-group tumors in exponential growth (200 to 800 mg range). The conversion of the T-C values to $\log_{10}$ cell kill is possible because the Td for tumors regrowing after treatment is approximately the same as that for untreated control mice. The net $\log_{10}$ kill value normalizes the efficacy data for tumor growth during treatment regimens of varied duration and provides an estimate of whether an actual regression of the tumor occurred. Positive values indicate that an actual reduction of tumor burden occurred. Negative values indicate the tumor actually grew (although possibly more slowly) during treatment. Tumor-free survivors were excluded from these calculations.

Acetyldinaline was suspended in 0.5% aqueous methyl cellulose and administered orally at various dosages in 0.5 mL volumes. Docetaxel was dissolved in 5% ethanol, 5% Tween 80, 90% aqueous saline and administered intravenously at various dosage levels in 0.2 mL injections.

The animals were divided into groups of 10 animals each. One group served as controls and received vehicle alone, with no drug treatments. Two groups received oral doses of acetyldinaline alone at a specified level (as shown in Table 3) of active drug (30 mg/kg and 60 mg/kg). The acetyldinaline was administered daily on Days 11-13 (Day 0 being when the tumor was implanted), Days 15-17, and Days 19-20. Three groups received docetaxel alone at doses of 3.5, 5.6, and 9.0 mg/kg. Two groups received acetyldinaline at the recited doses, in combination with 3.5 mg/kg of docetaxel, and another two groups received acetyldinaline at the recited doses in combination with docetaxel at 5.9 mg/kg, and the final two groups received acetyldinaline at the recited doses, in combination until 9.0 mg/kg of docetaxel.

RESULTS AND CONCLUSION

The antitumor effects that are produced when CI-994 is used in combination with docetaxel are shown in Table 3. The MTD of CI-994 was 60 mg/kg/day. This dose produced a 40% complete tumor response rate, and a 10% partial tumor response. The tumor growth delay for the tumors that did not completely respond to CI-994 was 4.9 days. This delay represents a net tumor cell kill of -0.3 $\log_{10}$. Thirty percent (30%) of the mice were tumor free when the study ended 105 days after the last CI-994 treatment. CI-994 at 30 mg/kg/day (50% its MTD) produced a 20% partial tumor response, but no complete tumor responses. The tumor growth delay produced by this dose 4.6 days, which represents a net tumor cell kill of -0.5 $\log_{10}$. The MTD of docetaxel was 9 mg/kg/day. This dose produced 30% partial tumor responses, but no complete tumor responses. The tumor growth delay produced by docetaxel at its MTD was only 3.3 days. This represents a net tumor cell kill of -0.2 $\log_{10}$.

While the agents individually produced only modest antitumor efforts, good antitumor activity was observed with all combinations of CI-994 and docetaxel. CI-994 could be given at its MTD with docetaxel at its MTD. This dose combination produced 40% CRs, 30% PRs, and a T-C of 15.7 days. This growth delay represents a net tumor cell kill of +0.2 $\log_{10}$. Twenty percent (20%) of the treated animals were tumor free when the study ended on Day 105.

Better antitumor activity was seen when CI-994 was given at its MTD with docetaxel at 62% of its MTD. This combination produced a 70% complete tumor response rate, a 100% partial response rate, a T-C of 12 days, and 100% of the mice were still tumor free when the study ended after 105 days.

These results indicate the antitumor activity is greater than additive when CI-994 is given at its MTD the same time as docetaxel at its MTD or lower. Indeed, based on the tumor response parameters of PR, CR, TF, and tumor growth delay, these results indicate the antitumor activity of the combination is greater than additive when CI-994 is used at its MTD and docetaxel is used at 62% of its MTD.

Table 3. Antitumor Effect of CI-994 in Combination With Docetaxel Against LC12 Carcinoma

| CI-994 | | Docetaxel | | Non-specific | % Weight | Antitumor Effect | | | | |
|---|---|---|---|---|---|---|---|---|---|---|
| Dose[a] | Schedule | Dose[a] | Schedule | Deaths | Change[b] | CR[c] | PR[d] | T-C[e] (+) | Net $Log_{10}$ Kill[f] | Tumor Free[g] |
| Vehicle | Days 11-13,15-17,19-21 | Vehicle | Days 11,15,19 | 0/10 | +5.2 | -- | -- | -- | -- | 0/10 |
| 30.0 | Days 11-13,15-17,19-21 | -- | -- | 0/10 | -5.5 | 0/10 | 2/10 | 4.6 | -0.5 | 0/10 |
| 60.0 | Days 11-13,15-17,19-21 | -- | -- | 0/10 | -10.5 | 4/10 | 1/10 | 4.9 | -0.3 | 3/10 |
| -- | -- | 3.5 | Days 11,15,19 | 0/10 | -7.3 | 0/10 | 0/10 | -1.7 | -- | 0/10 |
| -- | -- | 5.6 | Days 11,15,19 | 1/10 | -11.7 | 0/10 | 1/10 | -0.5 | -- | 0/10 |
| -- | -- | 9.0 | Days 11,15,19 | 1/10 | -16.7 | 0/10 | 3/10 | 3.3 | -0.2 | 0/10 |
| 30.0 | Days 12-14,16-18,20-22 | 3.5 | Days 11,15,19 | 1/10 | -13.5 | 2/10 | 1/10 | 4.5 (2.9) | -0.3 | 2/10 |
| 60.0 | Days 12-14,16-18,20-22 | 3.5 | Days 11,15,19 | 1/10 | -26.7 | 4/10 | 2/10 | 11.4 (3.2) | 0.0 | 4/10 |
| 30.0 | Days 12-14,16-18,20-22 | 5.9 | Days 11,15,19 | 0/10 | -17.8 | 4/10 | 2/10 | 6.3 (4.1) | -0.2 | 3/10 |
| 60.0 | Days 12-14,16-18,20-22 | 5.9 | Days 11,15,19 | 0/10 | -24.9 | 7/10 | 1/10 | 12.1 (4.4) | 0.1 | 7/10 |
| 30.0 | Days 12-14,16-18,20-22 | 9.0 | Days 11,15,19 | 0/10 | -17.8 | 3/10 | 2/10 | 15.4 (7.9) | 0.2 | 2/10 |
| 60.0 | Days 12-14,16-18,20-22 | 9.0 | Days 11,15,19 | 1/10 | -23.8 | 4/10 | 3/10 | 15.7 (8.2) | 0.2 | 2/10 |

[a]  Dose is in mg/kg/injection. The vehicle was composed of 0.5% hydroxypropylmethyl cellulose and 0.2% Tween-80 in water. The vehicle for docetaxel was composed of 0.5% EtOH/0.5% Tween-80/90% water.

[b]  A weight loss is the percent weight loss seen during treatment; the percent weight gain is the weight increase seen at the end of treatment.

[c]  Complete response is defined as a 100% reduction of initial tumor mass.

[d]  Partial response is defined as at least a 50% reduction of initial tumor mass.

[e]  T-C is the difference in days for the treated and control tumors to reach 750 mg. The values in parenthesis represent the T-C values for an additive antitumor effect. All tumor free survivors are excluded from T-C calculations.

[f]  Net $log_{10}$ tumor cell kill was calculated from the T-C value.

[g]  Tumor free represents the mice that had an undetectable tumor when the study ended on Day 105.

EXAMPLE 2

The combination of CI-994 plus docetaxel can be evaluated in mouse colon carcinoma cells (recombinant 26:10 cells), and the data was analyzed according to the Chou and Talalay program which establishes that the combination is synergistic.

Mouse colon carcinoma cells are seeded into 96-well culture plates in RPMI 1640 culture media supplemented with 20% fetal calf serum and 10 μg/mL of insulin. Various concentrations of CI-994 and docetaxel are added together 24 hours after cells were initially seeded into the culture plates and allowed to attach. The effect of CI-994 and docetaxel alone and in combination on colon carcinoma proliferation is determined after 96 hours of incubation at 37°C using the SRB assay (Skehan P, Stoneng R, Scudiero D, et al. New colorimetric cytotoxicity assay for anticancer-drug screening. *J. Natl. Cancer Inst.*, 1990;82:1107-1112). The combination chemotherapy data is analyzed using the Biosoft program, "Dose Effect Analysis with Microcomputers for IBM PC," which is a standard program for quantifying synergism, additivism, and antagonism among anticancer agents, and is based on the median-effect principle of mass-action law using an enzyme kinetic system model described by Chou and Talalay. Plots of fraction affected (Fa) versus combination index (CI) are called Fa-CI plots. These plots indicate synergism, additivity, or antagonism of 2 drugs at various effect levels in a mixture that is serially diluted. If several mixture are made, it is possible to estimate the optimal combination ratio for maximal synergy. Different effect levels usually give different degrees of synergism, additivism, or antagonism. CI values <1 indicate synergism; CI values >1 indicate antagonism and values that hover around 1 as a straight line indicate additivity.

EXAMPLE 3

Clinical Evaluation of 2-Component Combination Therapy

This is a multicenter, open-label Phase 1 study of CI-994 given in combination with docetaxel to patients with advanced solid tumors.

The objectives of this study are to determine the (1) MTD, (2) recommended Phase 2 dose, (3) pharmacokinetics, (4) safety profile, and (5) to observe for antitumor activity of CI-994 when given in combination with docetaxel to patients with advanced solid tumors. The primary efficacy endpoint is the attainment of either a PR or a CR. Secondary endpoints include time to PR or CR, duration of PR or CR, and survival.

Docetaxel is administered as an intravenous infusion at 3-week intervals during the treatment course, using an initial dose of 75 mg/m$^2$. CI-994 is administered orally as a daily dose for 21 days of a 28-day course, beginning on Day 1. Patients may receive subsequent courses of treatment based on individual tolerance and response to therapy. Patients whose disease does not respond or who develop intolerable adverse events are discontinued from study treatment.

The initial dose level of CI-994 is 4 mg/m$^2$. A minimum of three patients will be treated at each dose level. Dose levels are increased by 2 mg/m$^2$ until the MTD is reached. Ten additional patients are to be treated at the dose level recommended for Phase 2 studies, which is expected to be the MTD or one dose level below the MTD.

Once a patient begins study treatment, the addition of other cancer treatment will confound the assessment of safety and efficacy and therefore is not allowed. This restriction precludes the addition of systemic cytotoxic, hormonal, immunologic, or other biologic agents while the patient is in the treatment phase of this protocol. Patients who require palliative radiotherapy while on study are generally considered to have progressive disease and, unless compelling information exists to the contrary, are to be discontinued from study medication. Patients who develop new brain metastases while on study *may* have treatment interrupted to receive a course of cranial irradiation, then be restarted on study medication after a recovery period of at least 1 week.

Antiemetics may be used at the investigator's discretion for prevention and/or treatment of nausea or vomiting. Every effort should be made to ensure nausea and vomiting is controlled, as these conditions may preclude a patient from taking or absorbing the oral doses of CI-994.

Colony-stimulating factors may be used at the investigator's discretion to treat episodes of severe myelosuppression that are complicated by infection, but should otherwise not be used to support low blood counts or to maintain dose intensity.

If criteria are met for a CR, administer 2 additional courses of treatment beyond confirmation of the CR and then completely reassess the patient's disease state. If the patient is considered to be clinically free of disease at that time, discontinue the docetaxel and continue to administer CI-994 for 3 additional months, using the same dose and schedule (3 weeks on/1 week off). At that time, again completely reassess the patient's disease state. If the patient is still in CR, the investigator must evaluate the risks and potential benefits of continuing CI-994 treatment.

**Treatment Courses**

A treatment course consists of docetaxel given intravenously on Day 1 of a 28-day course *plus* CI-994 administered daily orally, beginning on Day 1, for 21 days of a 28-day course. Courses are to be repeated on Day 29 if there has been adequate recovery from adverse events and myelosuppression, defined as nonhematologic parameters of Grade ≤1, platelet count ≥100,000/µL, and absolute neutrophil count ≥1500/µL. Subsequent courses may be delayed by weekly intervals up to 3 weeks. If recovery has not occurred by Day 50, the patient is to be discontinued from study medication.

**Docetaxel Dosing**

The initial dose of docetaxel in each course is about 70 to 80 mg/m$^2$, given as a 1 to 2 hour intravenous infusion. Dose adjustments may be required during a treatment course. Follow the manufacturer's recommendations for information regarding preparation and administration.

**CI-994 Dose Levels**

CI-994 doses are calculated based on body surface area (BSA) and must then be rounded to the closest available capsule strength. CI-994 is available in capsule strengths of 2.5, 5, and 25 mg. Doses may be taken without regard to meals.

The initial CI-994 dose level is 4 mg/m$^2$. Subsequent dose levels will be increased (or decreased if necessary) by a fixed increment of 2 mg/m$^2$ until the MTD is determined. Individual patients may not receive dose escalations of gemcitabine or CI-994 in subsequent courses. Patients may receive a lower dose of CI-994 in a subsequent course if dose-limiting toxicities were experienced.

Three new patients will be assessed at each new dose level. The minimum time that these patients must be followed is 4 weeks before a new dose level may be opened (unless treatment was interrupted earlier and the patient is recovering from adverse events). If none of these three patients experience a dose-limiting toxicity, the next higher dose level will be opened. If one patient develops a dose-limiting toxicity, three more patients will be enrolled at that dose level. If ≥2 of 6 patients experience a dose-limiting toxicity at the same level, that dose level will be considered the MTD.

An assessable patient is defined as one who received 3 weekly doses of gemcitabine plus at least 80% of the CI-994 doses (≥17 doses), or a patient whose treatment course was discontinued early or was noncompliant (<17 doses) due to treatment-related adverse events. A patient who took fewer than 17 doses of CI-994 or did not complete the treatment course because of nontreatment-related reasons (e.g., missed appointments, ran out of CI-994 supplies, developed a coexisting medical condition that rendered the patient unable to swallow capsules, developed rapidly progressing disease) is not considered to be an assessable patient for the tolerability of that dose level.

Patients should be encouraged to take their CI-994 dose at approximately the same time each day. However, a variance of up to 12 hours either way is allowed for any given dose, rather than miss a day's dose. If a patient misses a day's dose entirely, they must be instructed not to 'make it up' the next day. If a patient vomits anytime after taking a dose of CI-994, they must be instructed not to 'make it up', but to resume subsequent doses the next day as prescribed.

On Days 1, 8, and 15, the CI-994 dose should be given 2 hours before the gemcitabine dose, to ensure maximal absorption in the event the patient develops vomiting following the gemcitabine dose.

Once the MTD is determined, 10 additional patients will be treated at the Phase 2 dose level, which is expected to be the MTD or one dose level below the MTD.

**Dose Adjustments During a Course**

Continuation of docetaxel and CI-994 during a course is dependent on patient tolerance and hematologic parameters. Reduced doses of docetaxel may be required on Days 8 and 15, as recommended by the manufacturer. The dose of CI-994 is not to be increased or decreased during a treatment course, although early termination may be required as described below. If both study medications must be stopped before a course is completed, do not complete that course, but instead follow the patient for recovery, then start another course using a reduced dose of CI-994.

The decision to discontinue CI-994 dosing during a course is based on adverse events or hematology results at any time. Example: A patient has a platelet count on Day 11 of 45,000/µL. Instruct the patient to stop taking CI-994 capsules (and to return all study medication containers to the site). Obtain another CBC on Day 15. If the platelet count on Day 15 is 50,000 to 99,000/µL, administer 75% of the calculated docetaxel dose but do not reinstitute CI-994 dosing. If the platelet count on Day 15 remains below 50,000/µL, do not retreat with gemcitabine. Consider this course to be terminated and follow the patient for recovery. In either case, the patient may receive a subsequent treatment course using the same initial dose of docetaxel and a CI-994 dose that has been reduced by 2 mg/m$^2$.

**Drug Formulation and Stability**

Docetaxel is to be obtained by the site from commercial sources. Follow the manufacturer's recommendation for preparation, administration, stability, and storage conditions.

CI-994 is formulated in identically appearing gelatin capsules containing 2.5, 5, or 25 mg of study medication, plus inactive ingredients of lactose, cornstarch, and talc or polyethylene glycol 6000. Store at controlled room temperature.

The foregoing data establish an unexpectedly favorable interaction between acetyldinaline in combination with docetaxel. Accordingly, this invention provides a method of treating susceptible neoplasms comprising administering acetyldinaline in a regimen together with docetaxel. The combination generally will include each active ingredient packaged separately, thereby avoiding any interaction between the agents prior to administration. If desired, the individually packaged drugs can be placed in a single carton as a kit, thereby providing convenience to the attending physician or medical attendant. The susceptible neoplasms to be treated according to this invention include solid tumors, especially advanced solid tumors and nonsmall cell lung cancer, as well as renal cell cancer, pancreatic cancer, head and neck cancer, ovarian cancer, myeloma, prostate cancer, and breast cancer.

**Claims**

1.   A combination comprising acetyldinaline and docetaxel.

2.   A combination of antineoplastic agents comprising an antitumor amount of acetyldinaline and an antitumor amount of docetaxel.

3.   The combination according to Claim 1 or Claim 2, wherein acetyldinaline or a pharmaceutically acceptable salt or hydrate thereof is formulated as a capsule.

4.   The combination according to any one of Claims 1 to 3, wherein docetaxel or a pharmaceutically acceptable salt or hydrate thereof is formulated as a sterile solution for intravenous infusion.

5.   The combination according to any one of Claims 1 to 4, wherein said docetaxel is docetaxel trihydrate monohydrochloride.

6.   Use of acetyldinaline or a pharmaceutically acceptable salt or hydrate thereof in combination with docetaxel or a pharmaceutically acceptable salt or hydrate thereof in the manufacture of a medicament for the curative, palliative or prophylactic treatment of cancer.

7.   Use of a combination according to any one of Claims 1 to 5 in the manufacture of a medicament for the curative, palliative or prophylactic treatment of cancer.

8.   Use according to Claim 6 or Claim 7, wherein the cancer is:

     a) non-small cell lung cancer ;
     b) breast cancer;
     c) prostate cancer; or
     d) locally advanced (nonresectable Stage II or Stage III) or metastatic (Stage IV) adenocarcinoma of the pancreas.

9.   A kit comprising acetyldinaline in one compartment and docetaxel in a second compartment.

10.  Products comprising acetyldinaline or a pharmaceutically acceptable salt or hydrate thereof and docetaxel or a pharmaceutically acceptable salt or hydrate thereof for use as a pharmaceutical.

11.  Products comprising acetyldinaline or a pharmaceutically acceptable salt or hydrate thereof and docetaxel or a pharmaceutically acceptable salt or hydrate thereof for use in the manufacture of a medicament for the curative, palliative or prophylactic treatment of cancer.

12. Products comprising acetyldinaline or a pharmaceutically acceptable salt or hydrate thereof and docetaxel or a pharmaceutically acceptable salt or hydrate thereof as a combined preparation for simultaneous, separate or sequential use in the curative, palliative or prophylactic treatment of cancer.

**Patentansprüche**

1. Kombination, die Acetyldinalin und Docetaxel umfasst.

2. Kombination von Antineoplastika, die eine gegen Tumorbildung wirksame Menge von Acetyldinalin und eine gegen Tumorbildung wirksame Menge von Docetaxel umfasst.

3. Kombination nach Anspruch 1 oder Anspruch 2, wobei Acetyldinalin oder ein pharmazeutisch akzeptables Salz oder Hydrat desselben als Kapsel formuliert ist.

4. Kombination nach einem der Ansprüche 1 bis 3, wobei Docetaxel oder ein pharmazeutisch akzeptables Salz oder Hydrat desselben als sterile Lösung für eine intravenöse Infusion formuliert ist.

5. Kombination nach einem der Ansprüche 1 bis 4, wobei das Docetaxel Docetaxeltrihydratmonohydrochlorid ist.

6. Verwendung von Acetyldinalin oder einem pharmazeutisch akzeptablen Salz oder Hydrat desselben in Kombination mit Docetaxel oder einem pharmazeutisch akzeptablen Salz oder Hydrat desselben zur Herstellung eines Medikaments zur kurativen, palliativen oder prophylaktischen Behandlung von Krebs.

7. Verwendung einer Kombination nach einem der Ansprüche 1 bis 5 zur Herstellung eines Medikaments zur kurativen, palliativen oder prophylaktischen Behandlung von Krebs.

8. Verwendung nach Anspruch 6 oder Anspruch 7, wobei der Krebs

    a) nicht-kleinzelliger Lungenkrebs,
    b) Brustkrebs,
    c) Prostatakrebs oder
    d) lokal fortgeschrittenes (nicht-reserzierbares Stadium II oder Stadium III) oder metastasierendes (Stadium IV) Adenokarzinom des Pankreas ist.

9. Kit, das Acetyldinalin in einer Abteilung und Docetaxel in einer zweiten Abteilung umfasst.

10. Produkte, die Acetyldinalin oder ein pharmazeutisch akzeptables Salz oder Hydrat desselben und Docetaxel oder ein pharmazeutisch akzeptables Salz oder Hydrat desselben umfassen, zur Verwendung als pharmazeutisches Mittel.

11. Produkte, die Acetyldinalin oder ein pharmazeutisch akzeptables Salz oder Hydrat desselben und Docetaxel oder ein pharmazeutisch akzeptables Salz oder Hydrat desselben umfassen, zur Verwendung bei der Herstellung eines Medikaments zur kurativen, palliativen oder prophylaktischen Behandlung von Krebs.

12. Produkte, die Acetyldinalin oder ein pharmazeutisch akzeptables Salz oder Hydrat desselben und Docetaxel oder ein pharmazeutisch akzeptables Salz oder Hydrat desselben als Kombinationszubereitung zur gleichzeitigen, getrennten oder aufeinanderfolgenden Verwendung bei der kurativen, palliativen oder prophylaktischen Behandlung von Krebs umfassen.

**Revendications**

1. Association comprenant de l'acétyldinaline et du docétaxel.

2. Association d'agents antinéoplasiques, comprenant une quantité antitumorale d'acétyldinaline et une quantité antitumorale de docétaxel.

**3.** Association suivant la revendication 1 ou la revendication 2, dans laquelle l'acétyldinaline ou un de ses sels ou hydrates pharmaceutiquement acceptables est formulée à l'état de capsule.

**4.** Association suivant l'une quelconque des revendications 1 à 3, dans laquelle le docétaxel ou un de ses sels ou hydrates pharmaceutiquement acceptables est formulé à l'état de solution stérile pour la perfusion intraveineuse.

**5.** Association suivant l'une quelconque des revendications 1 à 4, dans laquelle ledit docétaxel est le monochlorhydrate de trihydrate de docétaxel.

**6.** Utilisation d'acétyldinaline ou d'un de ses sels ou hydrates pharmaceutiquement acceptables en association avec le docétaxel ou un de ses sels ou hydrates pharmaceutiquement acceptables dans la production d'un médicament destiné au traitement curatif, palliatif ou prophylactique du cancer.

**7.** Utilisation d'une association suivant l'une quelconque des revendications 1 à 5, dans la production d'un médicament destiné au traitement curatif, palliatif ou prophylactique du cancer.

**8.** Utilisation suivant la revendication 6 ou la revendication 7, dans laquelle le cancer est :

a) un cancer pulmonaire non à petites cellules ;
b) le cancer du sein ;
c) le cancer de la prostate ; ou
d) un adénocarcinome localement avancé (stade II ou stade III non résécable) ou métastatique (stade IV) du pancréas.

**9.** Kit comprenant de l'acétyldinaline dans un compartiment et du docétaxel dans un second compartiment.

**10.** Produits comprenant de l'acétyldinaline ou un de ses sels ou hydrates pharmaceutiquement acceptables et du docétaxel ou un de sels ou hydrates pharmaceutiquement acceptables à des fins d'utilisation comme produits pharmaceutiques.

**11.** Produits comprenant de l'acétyldinaline ou un de ses sels ou hydrates pharmaceutiquement acceptables et du docétaxel ou un de ses sels ou hydrates pharmaceutiquement acceptables à des fins d'utilisation dans la production d'un médicament pour le traitement curatif, palliatif ou prophylactique du cancer.

**12.** Produits comprenant de l'acétyldinaline ou un de ses sels ou hydrates pharmaceutiquement acceptables et du docétaxel ou un de sels ou hydrates pharmaceutiquement acceptables sous forme d'une préparation combinée pour l'utilisation simultanée, séparée ou séquentielle dans le traitement curatif, palliatif ou prophylactique du cancer.